## Europäisches Patentamt
## European Patent Office
## Office européen des brevets

(19)

(11) Publication number: **0 031 228**
**B1**

(12)

# EUROPEAN PATENT SPECIFICATION

(45) Date of publication of patent specification: **20.07.83**

(51) Int. Cl.³: **A 61 B 10/00**

(21) Application number: **80304500.4**

(22) Date of filing: **12.12.80**

(54) Instrument for collecting tissue cells.

(30) Priority: **14.12.79 JP 173705/79**

(43) Date of publication of application:
**01.07.81 Bulletin 81/26**

(45) Publication of the grant of the patent:
**20.07.83 Bulletin 83/29**

(84) Designated Contracting States:
**DE FR GB**

(56) References cited:
**DE - A - 2 848 483**
**US - A - 2 839 049**

(73) Proprietor: **OLYMPUS OPTICAL CO., LTD.**
**43-2, 2-chome, Hatagaya Shibuya-ku**
**Tokyo 151 (JP)**

(72) Inventor: **Shimonaka, Hideki**
**4-11-4-402, Hashimoto**
**Sagamihara-Shi Kanagawa-ken (JP)**

(74) Representative: **Jones, Colin et al,**
**W.P. THOMPSON & CO. Coopers Building Church**
**Street**
**Liverpool L1 3AB (GB)**

Courier Press, Leamington Spa, England

# Instrument for collecting tissue cells

The invention relates to an instrument for collecting tissue cells, and more particularly, to such an instrument including an operating wire carrying on its free end a brush which is used to collect cells, and a sleeve tube which is adapted to projectably receive said brush and through which the operating wire is passed in a movable manner. In use, the sleeve tube is inserted into a channel of an endoscope which is adapted to receive the treatment instrument therein. The brush on the free end of the operating wire is then caused to project from the distal end of the tube for rubbing it against tissues of the interior of a physical body to collect cells therefrom.

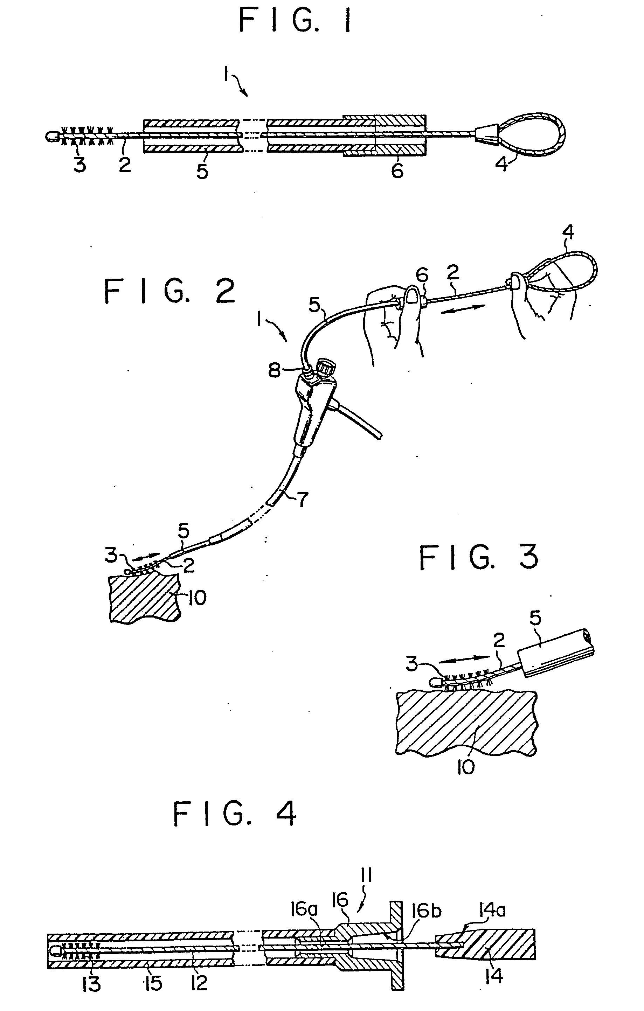

An example of a cell-collecting instrument having an operating wire which carries a collecting brush on its distal end and which is passed through a tube in a movable manner so that the brush can be projected from the tube is illustrated in Fig. 1 of the drawings. Specifically, the cell-collecting instrument 1 includes an operating wire 2 having a cell-collecting brush 3 mounted on its distal end and having a manipulator 4 at its proximate end. The brush 3 is received in a tube 5 in a movable manner, with a cap 6 fixedly connected to the proximate end of the tube. In use, the instrument 1 is inserted into a channel (not shown) of an endoscope 7 which is adapted to receive a treatment instrument through an inlet opening 8 thereof. While preventing the distal end of the collecting instrument 1 from projecting from the distal end of the endoscope 7, the latter is inserted into a coeliac cavity. During insertion, the distal end of the cell-collecting brush 3 is kept within the tube 5 to prevent the brush 3 from becoming contaminated inside the endoscope channel. Subsequently, the interior of the coeliac cavity is observed through the observation facility of the endoscope 7 until its distal end is brought to a desired location, whereupon the distal end of the tube 5 is caused to project from the distal end of the endoscope 7 until it is located opposite to cells which it is desired to collect, while holding the cap 6 by one hand. Thereafter, the cap 6 is held by the left hand while the manipulator 4 of the operating wire 2 is held by the right hand, as shown in Fig. 2, thus projecting the collecting brush 3 on the operating wire 2 out of the distal end of the tube 5. The operating wire may then be repeatedly drawn and pushed inward to cause the brush 3 to move back and forth as illustrated in exaggerated form in Fig. 3 to rub it against the wall 10 of the coeliac cavity, thereby collecting the desired cells. After collecting the cells, the brush 3 is once more retracted into the tube 7 to avoid contamination of the brush 3 inside the channel of the endoscope before the tube 5 is withdrawn from the endoscope 7. Finally, the brush is extracted to provide the desired cells for use.

With the conventional collecting instrument 1 described above, it will be appreciated that the cap of the tube 5 and the manipulator 4 of the operating wire 2 must be held by both hands to cause relative movement of the operating wire 2 with respect to the tube 5. When the magnitude of friction acting between the brush 3 and the cells to be collected is increased, the brush 3 cannot be moved and the tube 5 along moves with respect to the operating wire 2 which remains stationary, resulting in a failure to collect the cells.

It will be also noted in Fig. 2 that, because the cap 6 of the tube 5 and the manipulator 4 of the operating wire 2 must be held by both hands to cause a relative movement therebetween, there is a need for the help of a third party to hold the endoscope 7 or otherwise it must be left in an unstable condition.

Figs. 1 to 6 of US - A - 2 839 049 illustrate a cell-collecting brush on one end of a wire which is slidable in a tubular shield and whose other end may be clamped in a holder by means of a collet with the brush retracted into the tube. The tubular shield is not flexible so that it is not possible to use the instrument illustrated in Figs. 1 to 6 of US - A - 2 839 049 in conjunction with an endoscope. The cell-collecting instrument shown in Figs. 7 and 8 of US - A - 2 839 049 has a brush on a brush wire which is releasably clamped in an extended rigid arcuate handle. This instrument has no tubular shield into which the brush could be retracted.

In view of the foregoing, it is an object of the invention to provide a cell-collecting instrument which can be used with an endoscope and which is practical in use by eliminating the described disadvantage of the convention instrument, by allowing the operating wire to be joined to the tube in a secure manner when the brush on the wire projects from the distal end of the tube.

In accordance with the invention, there is provided an instrument for collecting cells of tissues from the interior of a physical body including an operating wire carrying a cell-collecting brush on its distal end, and a flexible tube through which the operating wire is passed in a movable manner, wherein, in use the tube is passed through a channel of an endoscope with the brush retracted within the tube so that the brush on the operating wire may be caused to project from the distal end of the tube to be rubbed against the tissues to collect cells therefrom after insertion of the endoscope into the physical body; characterized by a mechanism for connecting the operating wire to the flexible tube in a firm but detachable manner while the brush on the operating wire is projecting from the distal end of the flexible tube.

Accordingly, the collection of cells from tissues takes place by moving the entire collecting instrument back and forth while the operating wire is firmly attached to the tube. This avoids the likelihood that the brush may be pulled into the tube in the course of the collecting operation or that the brush remains immovable while the tube alone moves back and forth, and assures that the brush be rubbed against the tissues to collect cells therefrom.

The fact that the collection of cells takes place through a movement of the entire collecting instrument rather than as a result of a relative movement between the operating wire and the tube enables the collecting operation to be performed by utilizing one hand only, with the other hand being used to hold the endoscope. Hence, the need for the help of a third party is avoided, enabling a single person to operate the instrument.

The joining means is provided by a simple arrangement which utilizes the cap fixedly connected to the proximate end of the tube and a manipulator secured to the proximate end of the operating wire.

The invention is further described, by way of example, with reference to the drawings, in which:—

Fig. 1 is a longitudinal section of an example of a conventional cell-collecting instrument, already described;

Fig. 2 is a perspective view illustrating the manner of using the instrument shown in Fig. 1;

Fig. 3 is a larger-scale fragmentary view of the cell-collecting brush of Fig. 2 in use;

Fig. 4 is a longitudinal section of a cell-collecting instrument according to one embodiment of the invention;

Fig. 5 is a perspective view illustrating the manner of using the instrument shown in Fig. 4;

Fig. 6 is a fragmentary larger-scale section illustrating the manner of joining the operating wire of the tube during use as illustrated in Fig. 5;

Fig. 7 is larger-scale fragmentary view of the cell-collecting brush during its use as illustrated in Fig. 5; and

Fig. 8 is a longitudinal section of a cell-collecting instrument according to another embodiment of the invention.

Referring to Fig. 4, a cell-collecting instrument 11 according to one embodiment of the invention is constructed in generally the same manner as the known cell-collecting instrument 1 shown in Fig. 1, except for the construction of the cap 6 of the tube 5 and the manipulator 4 of the operating wire 2. Accordingly, corresponding parts are designated by reference numerals shown in Fig. 1 to which 10 is added, and will not be specifically described.

Thus, the cell-collecting instrument 11 includes a tube 15 having a bell-shaped cap 16 secured to the proximate end thereof. The cap 16 is formed with a through-opening 16a through which an operating wire 12 is passed. A manipulating member 14 is secured to the proximate end of the operating wire 12 and is formed with a tapered surface 14a which is adapted to fit in a corresponding tapered surface 16b formed in the cap 16. The manipulating member 14 is in the form of a shank having the tapered surface 14a formed on the exterior of its distal or front end. When the operating wire 12 is passed through the tube 15 and the tapered surface 14a of the manipulating member 14 fits in the tapered surface 16b formed in the cap 16 to join the manipulating member 14 securely with the tube 15, a cell-collecting brush 13 on the distal end of the operating wire 12 is fully projecting out of the distal end of the tube 15.

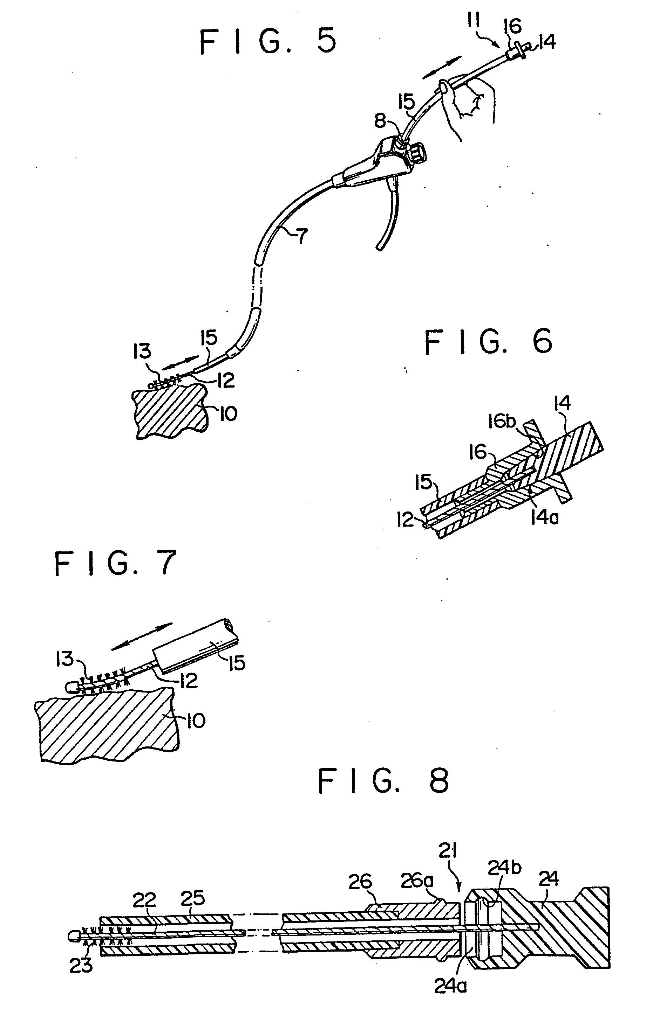

In use, the cell-collecting instrument 11 is initially inserted into a channel (not shown) of an endoscope 7 (see Fig. 5) which is adapted to receive a treatment instrument through an inlet opening 8 thereof with the brush 13 located within the distal end of the tube 15 as shown in Fig. 4 and not projecting out of the distal end of the endoscope 7. When the distal end of the endoscope 7 having the cell-collecting instrument 11 inserted therein is brought to a desired location within a coeliac cavity, the tube 15 is caused to project from the distal end of the endoscope 7, and the manipulating member 14 is driven into the cap 16 to engage the tapered surfaces 14a, 16b with each other to thereby securely connect the operating wire 12 to the tube 15, as shown in Fig. 6. Under this condition, the cell-collecting brush 13 projects fully out of the distal end of the tube 15, as shown in Figs. 5 and 7, for contact with a desired internal wall 10 of the coeliac cavity. At this time, by holding the proximate end of the tube 15 by one hand as illustrated in Fig. 5, it may be moved back and forth as indicated by a double-ended arrow, whereby the brush 13 secured thereto is rubbed against the cells of the wall 10 of the coeliac cavity in a positive manner, thus easily collecting such cells.

At this time, the endoscope 7 may be held by the other hand to stabilize it, and hence a reliable rubbing operation is facilitated. After the cells have been collected by the instrument 11, the cap 16 or the manipulating member 14 may be slightly turned relative to the other to separate the tapered surfaces 16b, 14a from each other before the instrument is pulled out of the coeliac cavity. When the cap 16 and the manipulating member 14 are disconnected from each other as shown in Fig. 4, the cell-collecting instrument 11 may be pulled out of the body in a conventional manner.

The cell-collecting instrument 21 of Fig. 8 is constructed in the same manner as the instrument 11 shown in Fig. 4 except for the construction of the cap 16 and the wire manipulating member 14. Accordingly, corresponding parts are designated by reference numerals of Fig. 4 to which 10 is added, and will not be specifically mentioned.

Thus, the cell-collecting instrument 21 includes a cap 26 which is cylindrical in configuration and which is provided with a rounded peripheral bead 26a on its external surface adjacent to the rear end thereof. The front end of the cap 26 is fixedly connected with the proximate end of a tube 25.

An operating wire 22 is provided with a manipulating member 24 of an elastic material which has a generally cylindrical configuration and a reduced diameter intermediate its ends in the manner of a pin cushion. The front end of the manipulating member 24 is formed with an opening 24a which is adapted to be fitted over the cap 26 of the tube 25. A peripheral groove 24b is formed in the inner surface of the opening 24a for fitting engagement with the peripheral bead 26a on the cap 26. The proximate end of the operating wire 22 is secured to the manipulating member 24 at the central portion of the bottom of the opening 24a.

In use, as the manipulating member 24 is fitted over the cap 26 and forced inwardly thereon, the resilience of the peripheral edge of the opening 24a permits such edge to be dilated sufficiently to enable the peripheral edge to slide over the peripheral bead 26a on the cap 26. When the peripheral groove 24b formed in the manipulating member 24 is engaged with the peripheral bead 26a, the resilience of the material of the manipulating member 24 permits the peripheral groove 24b to be restored into a firm fitting engagement with the bead 26a, whereby the manipulating member 24 is securely joined to the cap 26. Under this condition, a cell-collecting brush 23 projects fully out of the distal end of the tube 25, in the same manner as described above in connection with Fig. 4.

To release the manipulating member 24 from the cap 26, the latter is held stationary while the manipulating member 24 is strongly pulled rearwardly, whereupon the resilient edge of the opening 24a is again dilated to disengage the peripheral groove 24b from the bead 26a, thus separating the both members. It will be appreciated that, when the operating wire 22 is securely joined to the tube 25 in a detachable manner, a similar effect is achieved to that illustrated in Fig. 4 for securely joining the operating wire 12 to the tube 15.

## Claims

1. An instrument for collecting cells of tissues from the interior of a physical body including an operating wire (12 or 22) carrying a cell-collecting brush (13 or 23) on its distal end, and a flexible tube (15 or 25) through which the operating wire (12, 22) is passed in a movable manner, wherein, in use, the tube (15, 25) is passed through a channel of an endoscope (7) with the brush (13, 23) retracted within the tube (15, 25) so that the brush (13, 23) on the operating wire (12, 22) may be caused to project from the distal end of the tube (15, 25) to be rubbed against the tissues to collect cells therefrom after insertion of the endoscope (7) into the physical body; characterized by a mechanism (14a, 16b or 24b, 26a) for connecting the operating wire (12, 22) to the flexible tube (15, 25) in a firm but detachable manner while the brush (13, 23) on the operating wire (12, 22) is projecting from the distal end of the flexible tube (15, 25).

2. An instrument according to claim 1, in which a cap (16) is secured to the proximate end of the tube (15) and has an opening in its rear end which is defined by a tapered surface (16b), and a manipulating member (14) is secured to the proximate end of the operating wire (12) and has a tapered surface (14a) formed on its front peripheral portion which is adapted to be fitted into the tapered surface (16b) formed in the cap (16).

3. An instrument according to claim 1, in which said cap (26) is secured to the proximate end of the tube (25) and has a rounded peripheral bead or flange (26a) formed on its outer periphery adjacent to its rear end, and a manipulating member (24) of an elastic material is secured to the proximate end of the operating wire (22) and has an opening (24a) formed in its front end, with a peripheral groove (24b) formed in the inner surface of the opening (24a) for fitting engagement with the peripheral bead or flange (26a).

## Revendications

1. Instrument servant à recueillir des cellules tissulaires à l'intérieur d'un corps physique comprenant un fil métallique d'actionnement (12 ou 22) portant une brosse collectrice de cellules (13 ou 23) à son extrémité distale et un tube flexible (15 ou 25) à travers lequel on fait passer le fil d'actionnement (12, 22) de façon mobile, dans lequel, en service, on fait passer le tube (15, 25) à travers un conduit d'un endoscope (7), la brosse (13, 23) étant rétractée à l'intérieur du tube (15, 25) de sorte qu'on peut faire dépasser la brosse (13, 23) portée par le fil d'actionnement (12, 22), de l'extrémité distale du tube (15, 25) pour être frottée contre les tissus de manière à recueillir des cellules de ceux-ci après insertion de l'endoscope (7) dans le corps physique, caractérisé par un mécanisme (14a, 16b ou 24b, 26a) servant à relier le fil d'actionnement (12, 22) au tube flexible (15, 25) de façon ferme mais détachable tandis que la brosse (13, 23) portée par le fil d'actionnement (12, 22) dépasse de l'extrémité distale du tube flexible (15, 25).

2. Instrument selon la revendication 1, dans lequel un capuchon (16) est fixé à l'extrémité proximale du tube (15) et présente dans son extrémité postérieure une ouverture qui est définie par une surface tronconique (16b) et un organe de manipulation (14) est fixé à l'extrémité proximale du fil d'actionnement (12) et présente une surface tronconique (14a) formée sur sa partie périphérique antérieure et qui est conçue pour s'adapter dans la surface tronconique (16b) formée dans le capuchon (16).

3. Instrument selon la revendication 1, dans lequel le capuchon (26) est fixé à l'extrémité proximale du tube (25) et présente un bourrelet ou collet périphérique arrondi (26a) formé à sa périphérie extérieure auprès de son extrémité postérieure, et un organe de manipulation (24) en matière élastique est fixé à l'extrémité proximale du fil d'actionnement (22) et présente une ouverture (24a) formée dans son extrémité antérieure, une gorge périphérique (24b) étant formée dans la surface intérieure de l'ouverture (24a) pour l'adaptation au bourrelet ou collet périphérique (26a).

**Patentansprüche**

1. Instrument zur Entnahme von Gewebezellen aus dem Inneren eines physischen Körpers, mit einem an seinem distalen Ende eine Zellenentnahmebürste (13 oder 23) tragenden Betätigungsdraht (12 oder 22) und einem flexiblen Rohr (15 oder 25), durch das der Betätigungsdraht (12, 22) in bewegbarer Art hindurchgeführt ist, wobei bei der Benutzung das Rohr (15, 25) durch einen Kanal eines Endoskops (7) hindurchgeführt wird, während die Bürste (13, 23) innerhalb des Rohres (15, 25) zurückgezogen ist, so daß die Bürste (13, 23) an dem Betätigungsdraht (12, 22) veranlasst werden kann, aus dem Jistalen Ende des Rohres (15, 25) herauszuragen, um nach dem Einführen des Endoskops (7) in den physischen Körper gegen die Gewebe zur Entnahme von Zellen aus diesen gerieben zu werden, gekennzeichnet durch einen Mechanismus (14a, 16b oder 24b, 26a) zur Verbindung des Betätigungsdrahtes (12, 22) mit dem flexiblen Rohr (15, 25) in einer festen aber lösbaren Art, während die Bürste (13, 23) an dem Betätigungsdraht (12, 22) aus dem distalen Ende des flexiblen Rohrs (15, 25) herausragt.

2. Instrument nach Anspruch 1, bei dem eine Kappe (16) an dem proximalen Ende des Rohrs (15) befestigt ist und eine Öffnung in ihrem Rückende aufweist, die von einer sich verjüngenden Oberfläche (16b) gebildet ist, und ein Manipulierelement (14) an dem proximalen Ende des Betätigungsdrahtes (12) befestigt ist und eine an seinem vorderen Umfangsabschnitt gebildete sich verjüngende Oberfläche (14a) aufweist, die derart ausgebildet und angeordnet ist, daß sie in die in der Kappe (16) gebildete sich verjüngende Oberfläche (16b) eingepasst werden kann.

3. Instrument nach Anspruch 1, bei dem die Kappe (26) an dem proximalen Ende des Rohres (25) befestigt ist und einen an ihrem äußeren Umfang benachbart zu ihrem Rückende gebildeten abgerundeten Umfangswulst oder Flansch (26a) aufweist, und ein Manipulierelement (24) aus einem elastischen Material an dem proximalen Ende des Betätigungsdrahtes (22) befestigt ist und eine in seinem vorderen Ende gebildete Öffnung (24a) aufweist, wobei eine Umfangsnut (24b) in der inneren Oberfläche der Öffnung (24a) zum Paßeingriff mit dem Umfangswulst oder Flansch (26a) gebildet ist.

# FIG. 1

# FIG. 2

# FIG. 3

# FIG. 4

F I G. 5

F I G. 6

F I G. 7

F I G. 8